# EUROPEAN PATENT SPECIFICATION

(11) **EP 0 923 540 B1**
(45) Date of publication and mention of the grant of the patent: **27.03.2002**
(21) Application number: 97939711.4
(22) Date of filing: 29.08.1997
(51) Int. Cl.: C07C 249/16, C07D 237/24

(54) **SYNTHESIS OF A HYDRAZONE BETA-KETO ESTER BY THE REACTION WITH A DIAZO ESTER**
HERSTELLUNG VON EINEM HYDRAZON-BETA-KETOESTER DURCH REAKTION MIT EINEM DIAZOESTER
SYNTHESE D'UN HYDRAZOESTER BETA-CETO PAR REACTION AVEC UN DIAZOESTER

(30) Priority: 30.08.1996 US 24963 P; 10.04.1997 US 43455 P
(43) Date of publication of application: 23.06.1999
(73) Proprietor: Monsanto Technology LLC, St. Louis, Missouri 63167 (US)
(72) Inventor: SHAH, Ajit, S., St. Louis, MO 63146 (US); CLARK, Jerry, D., Alton, IL 62002 (US); MA, Yinong, St. Louis, MO 63146 (US); PETERSON, James, C., Manchester, MO 63021 (US); PATELIS, Lefteris, Creve Coeur, MO 63141 (US)
(74) Representative: Bosch, Henry
(86) International application number: US9715345
(87) International publication number: WO9808807

(56) References cited:
- US-A- 4 707 181
- US-A- 4 962 199
- US-A- 5 189 163
- HOLMQUIST, CHRISTOPHER. R. ET AL: "A selective method for the direct conversion of aldhydes into beta-keto esters with ethyl diazoacetate catalyzed by tin(II) chloride." JOURNAL OF ORGANIC CHEMISTRY., no. 54, 1989, pages 3258-3260, XP002049917
- ABBASS, IKHLASS M. ET AL: "Synthesis of pyrazolo [4,5]pyridazine and isoxazolo[3,4d]pyridazine derivatives" ARCH. PHARM. RES. , vol. 15, no. 3, 1992, pages 224-228, XP002049918

## Description

### 1. Field of the Invention

The present invention relates generally to a process for making pyridazinone derivatives and a hydrazone aldehyde precursor.

### 2. Description of Related Art

Certain carboxy substituted 4-oxo-1,4-dihydropyridazines and carboalkoxy substituted 4-oxo-1,4-dihydropyridazines are known in the art to have plant gametocidal activity and plant growth regulatory activity. Current methods for producing the above compounds typically use expensive raw materials and/or result in low yields of product. For example, U.S. Patent Nos. 4,707,181; 5,026,880; and 4,732,603 disclose preparing the above compounds by reacting a 2-phenylhydrazono-3-oxoglutarate with an organic acid chloride in the presence of a Grignard reagent (isopropyl magnesium chloride). The Grignard reagents are expensive and low yields are obtained. U.S. Patent Nos. 5,189,163 and 5,010,192 also disclose using expensive raw materials, such as methyl-3-oxopentanoate, and result in low yields.

The present invention provides a more efficient process for making pyridazinone derivatives which results in higher yields of product and uses lower cost raw materials, hydrazone aldehyde and diazo acetate.

Hydrazone aldehydes are useful as precursors in the production of the carboxy substituted 4-oxo-1,4-dihydropyridazines and carboalkoxy substituted 4-oxo-1,4dihydropyridazines mentioned above. Such hydrazone aldehydes, such as 4-chlorophenylhydrazone aldehyde (ethanedial, mono[(4-chlorophenyl)hydrazone]), have typically been made by batch processes, wherein one reactant is added to a reactor containing the other reactant. However, such batch processes lead to a lower payload (2-5%). An increase in the payload above 5% made the reaction slurry difficult to mix and thus resulted in by-product formation. In addition, the water content of the resulting solution is about 80% in a batch process, making the solution difficult to wash and handle, and causing filtration of the product to be very slow. The high water content also leads to very long drying times of the product.

An improved process to produce hydrazone aldehydes having a decreased water content would lead to a solution which is easier to handle, and decreased filtration and drying times of the product.

### SUMMARY OF THE INVENTION

The present invention relates to the synthesis of a hydrazone β-keto ester by the reaction of a diazo ester with a hydrazone aldehyde in the presence of a Lewis acid. In a preferred embodiment, the hydrazone β-keto ester is then converted into a pyridazinone compound by the reaction with an alkyl acid chloride in the presence of a base, followed by acidification.

More particularly, the present invention relates to the synthesis of a hydrazone β-keto ester having the general Formula III: 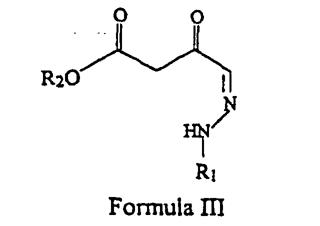 by reacting a diazo ester with a hydrazone aldehyde in the presence of a Lewis acid.

The hydrazone β-keto ester can then be reacted with an alkyl acid chloride in the presence of a base, followed by acidification to produce pyridazinone compounds having the general Formula I: 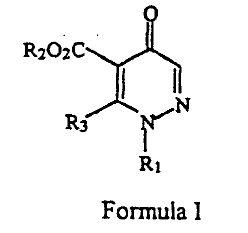 or the general Formula II:

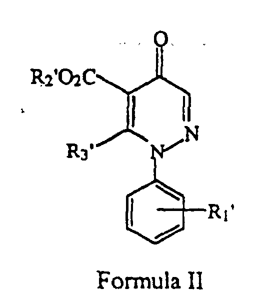

### DESCRIPTION OF ILLUSTRATIVE EMBODIMENTS

The present invention relates generally to a process for making pyridazinone derivatives and preferably to a process for making a compound of Formula I: 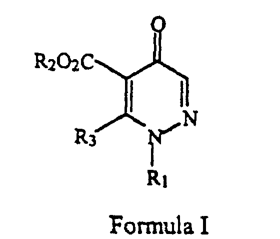 wherein R₁ is an alkyl, cycloalkyl, aryl or heteroaromatic group; R₂ is an alkyl group; and R₃ is an alkyl or phenyl group. Alkyl groups useful in the processes of the present invention include straight-chain, branched-chain or cyclic alkyl groups having between about 1 and 12 carbon atoms. Preferably the alkyl groups have from 1 to about 5 carbon atoms. Aryl in a given case can be phenyl and can be substituted with one or more lower alkyl groups and/or one or more halogen atoms such as Cl, Br or F and/or lower alkoxy group. Heteroaromatic groups include: furanyl, thienyl, pyridyl, etc., and can be optionally substituted as described in the case of phenyl groups.

In a preferred embodiment, the present invention relates to the production of pyridazinone compounds of Formula II: 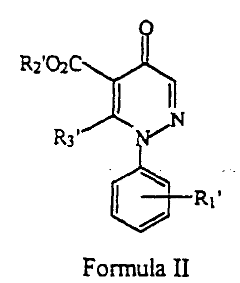 wherein R₁' is an alkyl and/or halo group, R₂' is an alkyl group and R₃' is an alkyl or phenyl group.

The pyridazinone compounds of Formula I and II have plant gametocidal activity and plant growth regulatory activity. In the process of producing the pyridazinone compounds of Formula I and II, a β-keto ester is produced by reacting a hydrazone aldehyde with a diazo ester in the presence of a Lewis acid. The β-keto ester has the general Formula III: 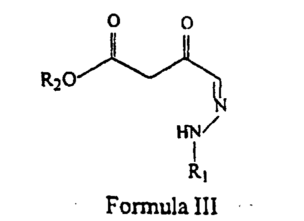 wherein R₁ and R₂ arc as previously described. β-Keto esters can also be used in the synthesis of pyrazoles, which are used to prepare drugs, dyes and crop protection agents. The reaction can be generally described as follows: 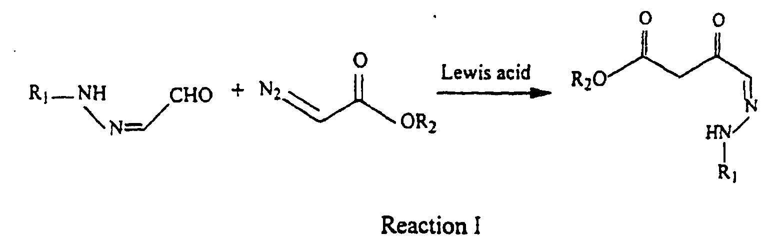 wherein R₁ and R₂ are as previously described.

The reaction of the hydrazone aldehyde and alkyl diazoester to produce a β-keto ester can be conducted in the presence of a solvent. Solvents useful in the reaction of hydrazone aldehyde with diazo ester include organic solvents such as toluene, cumene, benzene, ethylbenzene, diethyl ether, dibutyl ether, butyl ethyl ether, chlorobenzene, nitrobenzene, ortho-dichlorobenzene, and chlorinated hydrocarbons such as methylene chloride and dichloroethane.

Alkyl diazoesters used in Reaction I to produce the β-keto ester are commercially available, such as from Aldrich Chemical Co., or can be produced as disclosed in U.S. Patent Nos. 2,490,714; 2,691,649; and 2,691,650.

The hydrazone aldehyde can be added as a solid or in a slurry. Lewis acids suitable for use in this reaction have been previously described. For example, *see* Holmquist, C.R.; Roskamp, E.J. *J. Org. Chem.* **1989,** 54, 3258. Lewis acids which are suitable in this reaction include SnCl₂, ZnCl₂, ZrCl₄, AlCl₃, TiCl₄, SnCl₄, ZrCl₄ ^{.} (THF)₂, zeolites, SnCl₂ + triphenylmethyl chloride (TMSCI), and SnCl₂ + TMSCI. Mixtures of Lewis acids can also be used. Preferred Lewis acids are SnCl₂ and SnCl₄, and the Lewis acid should be present in a catalytic amount, typically between about 5 mol% and 25 mol%. The reaction is generally started at a temperature below room temperature, typically between about 0°C and about 25°C, and gradually allowed to warm to room temperature during the course of the reaction. The hydrazone aldehyde and diazo ester are typically added in approximately stoichiometric proportions, although ratios of hydrazone aldehyde to diazo ester of about 2:1 to about 1:2 can also be used.

The β-keto ester is then treated with a base and an alkyl acid halide, followed by acidification, to produce a compound of Formula I as shown below: 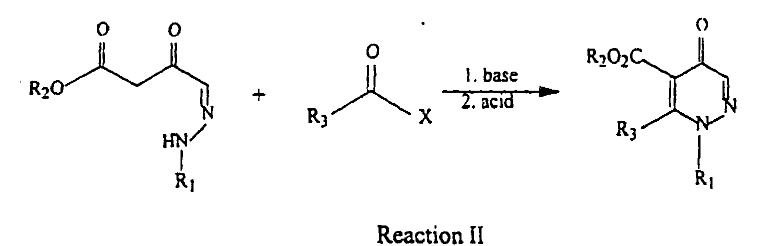 wherein R₁, R₂ and R₃ are as described previously, and X is a halide, preferably chloro or bromo.

Reaction II of the β-keto ester with an alkyl acid halide and base can also be conducted in the presence of a solvent as described above. The β-keto ester can be purified prior to use in this reaction or can be used directly from Reaction I without purification. Bases useful in this reaction are generally described below, and a preferred base is Ca(OH)₂. A preferred alkyl acid halide suitable in this reaction is propionyl chloride. Reaction II can also be conducted in the presence of an acylation catalyst, such as pyridine or a substituted pyridine, preferably 4-dimethylaminopyridine (DMAP), polymer-supported DMAP, or 4-(4-methyl-1,1-piperidinyl)pyridine. Acids useful in the acidification that follows are generally any acid known for such acidification, and the acid is preferably HCl, H₂SO₄ or H₃PO₄.

Reaction II is preferably conducted at a temperature between about 0 °C and about 40 °C. The acid halide and base are generally added in slight excess, such that the ratio of acid halide to b-keto ester or the ratio of base to b-keto ester is between about 1:1 to about 3:1. The acylation catalyst can be used in an amount between about 0 and 15 mol%, preferably between about 8 and 10 mol%.

Bases which can be used in the processes of the present invention include both organic and inorganic bases. Suitable inorganic bases include Group I and II metal hydrides, hydroxides and oxides, such as sodium hydride, calcium hydroxide, calcium oxide, barium hydroxide, barium oxide, magnesium hydroxide, magnesium oxide and the like. Suitable organic bases include both alkyl and aromatic bases such as alkyl, cycloalkyl and aryl amines, metallic amides and aromatic amines. Suitable alkyl and aryl amines include diethylamine, triethylamine, benzylamine, piperidine, piperazine, pyrrolidine and the like. Alkylamines, such as triethylamines, are preferred. Suitable metallic amides include sodium amide and lithium diisopropyl amide. Suitable aromatic amines (aromatic, nitrogen heterocylic compounds) include imidazole, methylimidazole, pyrazole, pyridine. pyrimidine, pyridazine and the like, preferably pyridines. Those skilled in the art will appreciate that other bases can be utilized in the process of the present invention.

A compound of the general Formula I can be converted to the acid and then the salt form using standard procedures, such as those disclosed in U.S. Patent No. 5,026,880. For example, the solid free acid can be made by treatment of the compound of Formula I with NaOH, followed by acidification with HCl: 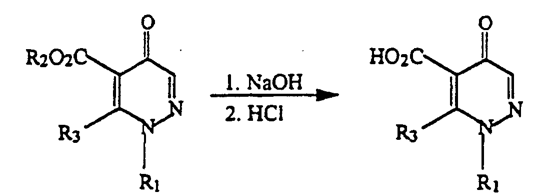 The potassium salt can subsequently be made by treatment of the free acid with KOH.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1

This example illustrates Reaction I, the production of a β-keto ester by reacting a hydrazone aldehyde and diazo ester in the presence of a Lewis acid.

To a slurry of 10.3 g (0.06 mol) of hydrazone aldehyde (ethanedial, mono[(4-chlorophenyl)hydrazone]) in 600 mL of methylene chloride at 25°C, 2.55 g (0.014 mol) of tin(II) chloride was added. Then, 6.78 g (.06 mol) of ethyldiazo acetate was added. The reaction was stirred for 18 h at 25°C. The reaction mixture was diluted with 100 mL of methylene chloride and to the resulting mixture was added 100 mL of water. After stirring for 5 minutes, the mixture was filtered through celite and the methylene chloride layer was separated and concentrated under reduced pressure to obtain a dark brown residue. The product, butanoic acid (4-[(4-chlorophenyl)-(1-hydrazinyl-2-ylidene)]-3-oxo-) ethyl ester, was purified by crystallization from ether and hexane as a yellow solid (8.20 g, 54.2 % yield), m.p. 100-100.5 °C.

### Example 2

This example illustrates Reaction II, wherein a β-keto ester is reacted with an alkyl acid halide to produce a pyridazinone compound having the general Formula I.

A solution of 0.5 g (1.87 mmol) of the β-keto ester produced in Example 8 in 10 mL of toluene was treated with 0.21 g (2.79 mmol) of Ca(OH)₂. The slurry was stirred for 2 h at 25°C. Propionyl chloride (0.25 g, 2.79 mmol) was then added slowly, and the reaction was stirred for 5h at 25 °C.

At this time 10 mL of water and 20 mL of 5N HCl was added, and the reaction was stirred for 30 minutes to solubilize the calcium salts.

The top toluene layer was separated (the bottom aqueous layer was discarded) and heated to 80°C for 2 h. After cooling excess solvent was removed under reduced pressure to yield 0.57 g of an amber solid. This product was 95% pure by ¹H NMR (99.8% yield). 180 mg of the crude product was purified by chromatography (silica gel, 50% ethyl acetate in hexane) to yield 160 mg of pure product, 4-pyridazinecarboxylic acid (2-(4-chlorophenyl)-3-ethyl-2,5-dihydro-5-oxo-) ethyl ester, as a yellow solid, m.p. 130-131°C.

### Example 3

This example illustrates the synthesis of a β-keto ester from hydrazone aldehyde and ethyl diazoacetate, shown in the reaction below:

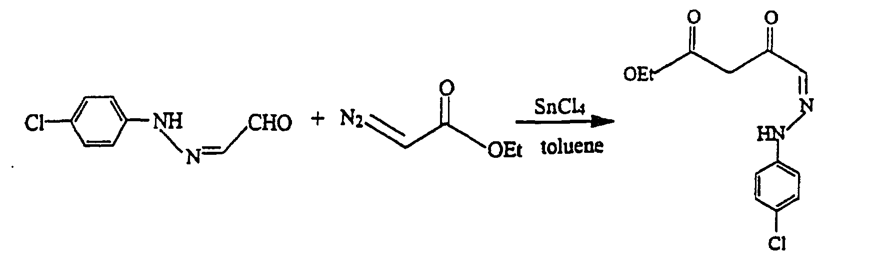

A dry reactor is charged with 162.2 g of a solution of 10.0 wt-% ethyl diazoacetate in toluene, and 20.0 g of [(4-chlorophenyl)hydrazono] acetaldehyde. The slurry is then cooled to 0°C.

To the cooled slurry is added 8.09 mL of 1.00 M tin (IV) chloride solution in toluene over a period of 80 min. The temperature of the slurry is maintained below 5°C during the addition.

The mixture is then allowed to warm to room temperature and is stirred for an additional 2 hours. The reaction is monitored by HPLC analysis. Chemical yield is about 67.5%. All of the hydrazone aldehyde is consumed.

### Example 4

This example illustrates the synthesis of a diketo ester from a β-keto ester, shown in the reaction below:

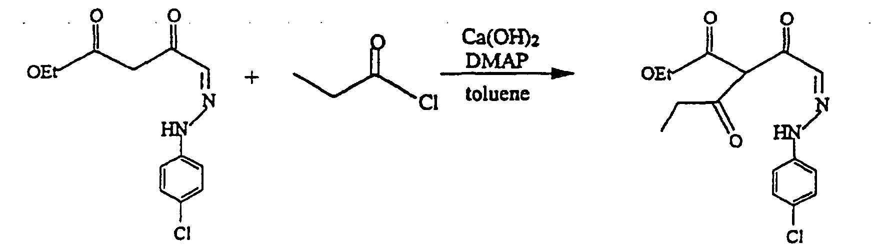

To the agitated crude reaction mixture at 22°C from Example 4, is added 8.45 g of calcium hydroxide. The reactor is then charged with 1.34 g of 4-dimethylaminopyridine (DMAP).

Propionyl chloride (9.81 g ) is then added over a period of 10 min. The temperature of the mixture increases from 28 to 32°C. The reaction is complete after the propionyl chloride is added based on HPLC analysis. The chemical yield is about 90%.

### Example 5

This example illustrates the synthesis of the ethyl ester from the diketo ester of Example 4, shown in the reaction below:

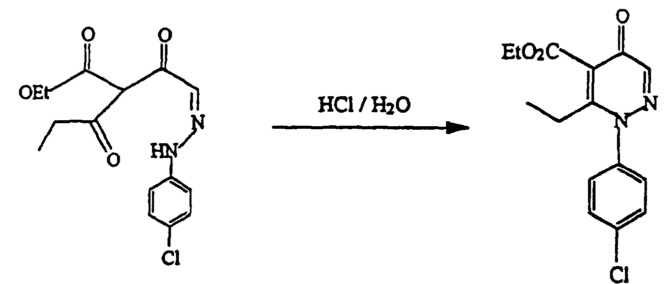

To the vigorously agitated crude-reaction mixture from Example 4 is added 68 mL of 1.2 N HCl (aq.). The biphasic system is heated to 80°C and this temperature is maintained for 1 hour. The reaction is complete based on HPLC analysis.

The contents are filtered at 80°C to remove insoluble materials that are suspended in the aqueous layer. The filtration is complete within 1 h. The aqueous layer is separated from the organic layer, and the organic layer (top) (191 g) contains the ethyl ester, and can then be used to produce the free acid. The aqueous layer (bottom) is discarded. The chemical yield is about 90%.

### Example 6

This example illustrates the synthesis of the free acid of the ethyl ester of Example 5 via saponification and acidification, shown in the reaction below:

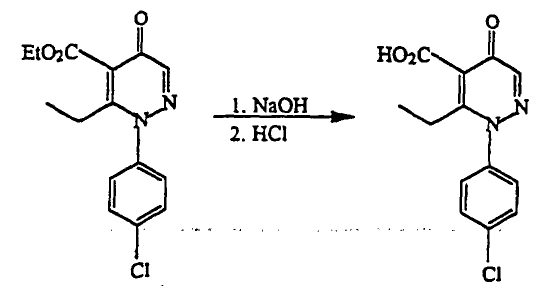

To the vigorously agitated reaction mixture from Example 7, is added 137 g of 15% w/w aqueous sodium hydroxide solution. The biphasic system is heated to 60°C and is stirred for 2 h. The layers are separated. The top organic phase is discarded. The bottom aqueous layer (165 g) is treated with 100 mL isopropanol.

To this mixture is added 50 mL of concentrated hydrochloric acid (37% wt) over 10 min. The resulting mixture is cooled to room temperature while stirring (30 min). The contents are filtered, and the filtered cake is washed with 50 mL of isopropanol. The chemical yield is about 95%. After air drying, 13 g of solid with greater than 95% purity of the free acid was isolated, as characterized by ¹H and ¹³C NMR.

## Claims

1. A process for the production of a hydrazone β-keto ester of Formula III: 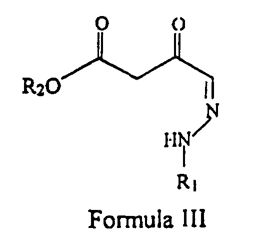 wherein R₁ is an alkyl, cycloalkyl, aryl or heteroaromatic group; and R₂ is an alkyl group; comprising contacting a diazo ester with a hydrazone aldehyde in the presence of a Lewis acid.

2. The process of Claim 1 wherein the diazo ester is an alkyl or benzyl diazo acetate.

3. The process of Claim 1 wherein the hydrazone aldehyde is ethanedial, mono[(4-chlorophenyl)hydrazone].

4. A process for the production of a pyridazinone compound of formula I: 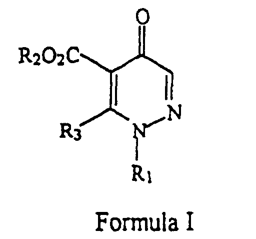 wherein R₁ is an alkyl, cycloalkyl, aryl or heteroaromatic group; R₂ is an alkyl group; and R₃ is an alkyl or phenyl group; comprising:
contacting a diazo ester with a hydrazone aldehyde in the presence of a Lewis acid to produce a hydrazone β-keto ester,
contacting the hydrazone β-keto ester with an alkyl acid halide in the presence of a base to form a diketo ester, and
contacting the diketo ester with an acid.

5. A process of claim 4 where said pyridazinone compound is of formula II: 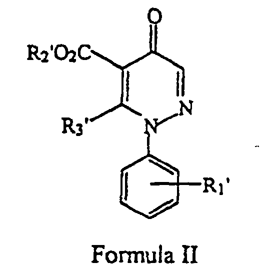 wherein R₁' is an alkyl and/or halo group, R₂' is an alkyl group and R₃' is an alkyl or phenyl group.

6. The process of Claim 5 wherein R₁' is a chloride, R₂' is ethyl, and R₃' is ethyl.

7. The process of Claim 5 wherein the diazo ester is an alkyl or benzyl diazo acetate.

8. The process of Claim 7 wherein the diazo ester is ethyl diazo acetate.

9. The process of Claim 8 wherein the diazo ester is isopropyl diazo acetate.

10. The process of Claim 4 wherein the hydrazone aldehyde is ethanedial, mono[(4-chlorophenyl)hydrazone].

11. The process of Claim 4 wherein the Lewis acid is a tin(II) or tin(IV) compound.

12. The process of Claim 11 wherein the Lewis acid is SnCl₂ or SnCl₄.

## Patentansprüche

1. Verfahren zur Herstellung eines Hydrazon-β-ketoesters der Formel III: 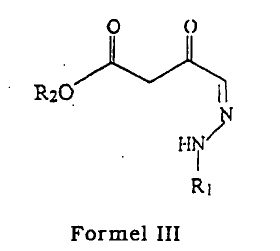 worin R₁ eine Alkyl-, Cycloalkyl-, Aryl- oder heteroaromatische Gruppe ist; und R₂ eine Alkylgruppe ist; umfassend das Kontaktieren eines Diazoesters mit einem Hydrazonaldehyd in Gegenwart einer Lewissäure.

2. Verfahren nach Anspruch 1, wobei der Diazoester ein Alkyl- oder Benzyldiazoacetat ist.

3. Verfahren nach Anspruch 1, wobei der Hydrazonaldehyd Ethandial, Mono[(4-chlorphenyl)hydrazon] ist.

4. Verfahren zur Herstellung einer Pyridazinonverbindung der Formel I: 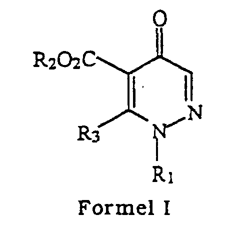 worin R₁ eine Alkyl-, Cycloalkyl-, Aryl- oder heteroaromatische Gruppe ist; R₂ eine Alkylgruppe ist; und R₃ eine Alkyl- oder Phenylgruppe ist; umfassend:
Kontaktieren eines Diazoesters mit einem Hydrazonaledyd in Gegenwart einer Lewissäure zur Erzeugung eines Hydrazon-β-ketoesters,
Kontaktieren des Hydrazon-β-ketoesters mit einem Alkylsäurehalogenid in Gegenwart einer Base zur Bildung eines Diketoesters, und
Kontaktieren des Diketoesters mit einer Säure.

5. Verfahren nach Anspruch 4, wobei die Pyridazinonverbindung der Formel II entspricht: 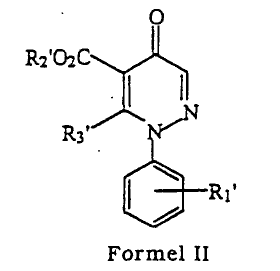 worin R₁' eine Alkyl- und/oder Halogengrupppe ist, R₂' eine Alkylgruppe ist, und R₃' eine Alkyl- oder Phenylgruppe ist.

6. Verfahren nach Anspruch 5, wobei R₁' ein Chlorid ist, R₂' Ethyl ist, und R₃' Ethyl ist.

7. Verfahren nach Anspruch 5, wobei der Diazoester ein Alkyl- oder Benzyldiazoacetat ist.

8. Verfahren nach Anspruch 7, wobei der Diazoester Ethyldiazoacetat ist.

9. Verfahren nach Anspruch 8, wobei der Diazoester Isopropyldiazoacetat ist.

10. Verfahren nach Anspruch 4, wobei der Hydrazonaldehyd Ethandial, Mono[(4-chlorphenyl)hydrazon] ist.

11. Verfahren nach Anspruch 4, wobei die Lewissäure eine Zinn(II)- oder Zinn(IV)-Verbindung ist.

12. Verfahren nach Anspruch 11, wobei die Lewissäure SnCl₂ oder SnCl₄ ist.

## Revendications

1. Procédé de production d'un hydrazone-β-cétoester de Formule III : 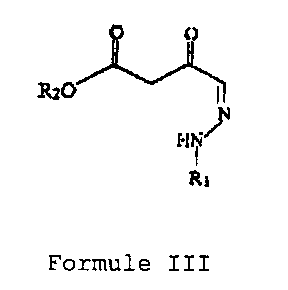 dans laquelle R₁ représente un groupe alkyle, cycloalkyle, aryle ou hétéroaromatique ; et R₂ représente un groupe alkyle ; comprenant la mise en contact d'un diazoester avec un hydrazone-aldéhyde en présence d'un acide de Lewis.

2. Procédé selon la revendication 1, dans lequel le diazoester est un diazoacétate d'alkyle ou de benzyle.

3. Procédé selon la revendication 1, dans lequel l'hydrazone-aldéhyde est l'éthanedial, mono[(4-chlorophényl)hydrazone].

4. Procédé de production d'un composé de pyridazinone de Formule I : 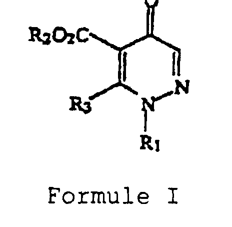 dans laquelle R₁ représente un groupe alkyle, cycloalkyle, aryle ou hétéroaromatique ; R₂ représente un groupe alkyle ; et R₃ représente un groupe alkyle ou phényle ; comprenant :
la mise en contact d'un diazoester avec un hydrazone-aldéhyde en présence d'un acide de Lewis pour produire un hydrazone-β-cétoester,
la mise en contact de l'hydrazone-β-cétoester avec un halogénure d'acide alkylique en présence d'une base pour former un dicétoester, et
la mise en contact du dicétoester avec un acide.

5. Procédé selon la revendication 4, dans lequel ledit composé de pyridazinone répond à la Formule II : 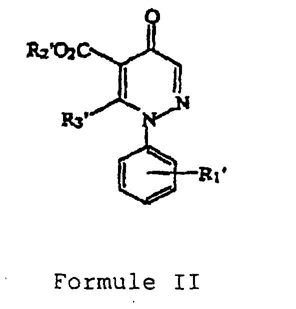 dans laquelle R₁' représente un groupe alkyle et/ou un atome d'halogène, R₂' représente un groupe alkyle et R₃' représente un groupe alkyle ou phényle.

6. Procédé selon la revendication 5, dans lequel R₁' représente un atome de chlore, R₂' représente un groupe éthyle et R₃' représente un groupe éthyle.

7. Procédé selon la revendication 5, dans lequel le diazoester est un diazoacétate d'alkyle ou de benzyle.

8. Procédé selon la revendication 7, dans lequel le diazoester est le diazoacétate d'éthyle.

9. Procédé selon la revendication 8, dans lequel le diazoester est le diazoacétate d'isopropyle.

10. Procédé selon la revendication 4, dans lequel l'hydrazone-aldéhyde est l'éthanedial, (mono[(4-chlorophényl)hydrazone].

11. Procédé selon la revendication 4, dans lequel l'acide de Lewis est un composé d'étain(II) ou d'étain(IV).

12. Procédé selon la revendication 11, dans lequel l'acide de Lewis est SnCl₂ ou SnCl₄.
